# EUROPEAN PATENT APPLICATION

(11) **EP 4 201 948 A1**
(43) Date of publication of application: **28.06.2023**
(21) Application number: 21306866.1
(22) Date of filing: 21.12.2021
(51) Int. Cl.: C07H 1/00, C07H 1/06, C07H 17/02

(54) **METHOD OF PREPARATION OF 1-(N-(QUINOLIN-2-YL)-(PHENYLAMINO)-1-DEOXY-BETA-D-GLUCOPYRANURONIC ACID DERIVATIVES**

(71) Applicant: ABIVAX, 75008 Paris (FR)
(72) Inventor: ROSE, Sébastien, 45300 PITHIVIERS (FR); MAGUEUR, Guillaume, 45300 PITHIVIERS (FR)
(74) Representative: Cabinet Nony

(57) **Abstract**

The present invention relates to a process of purification of a crude compound of formula (I) 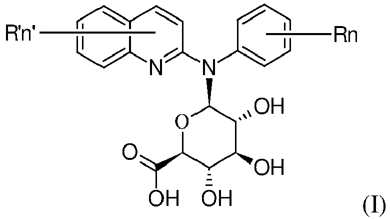 wherein it comprises an isolation step of said crude compound of formula (I) including: (i) a solubilization step of said crude compound of formula (I) with methanol followed by a precipitation step with water, and (ii) a drying step under a temperature inferior or equal to 30°C.

Herein is further provided a m manufacturing process for preparing a compound of formula (I).

## Description

### FIELD OF THE INVENTION

Herein is provided a new method for purifying 1-(N-(quinolin-2-yl)-(phenylamino)-1-deoxy-β-D-glucopyranuronic acid derivatives named herein after "quinolinyl-2-yl-phenylalamine glucuronide derivatives", in particular for purifying 1-(N-(8-chloro-quinolin-2-yl)-(4-tyrifluoromethoxy-phenylamino)-1-deoxy-β-D-glucopyranuronic acid. The provided process in more particularly suitable for industrial scale. Herein is also provided an improved process for the preparation of quinolinyl-2-yl-phenylalamine glucuronide derivatives, in particular of 1-(N-(8-chloro-quinolin-2-yl)-(4-tyrifluoromethoxy-phenylamino)-1-deoxy-β-D-glucopyranuronic acid, more adapted for industrial implementation.

### BACKGROUND OF THE INVENTION

WO 2016/135052 application describes the preparation and the use of quinoline-2-yl-phenylamine glucuronide derivative. Said glucuronide derivative is a N-glucuronide metabolite of chloro-N-[4-(trifluoromethoxy)phenyl]quinolin-2-amine, also named ABX464. Said compound is disclosed as being useful in the treatment or prevention of viral or retroviral infection and virus-related conditions, in particular AIDS or an AIDS-related condition or Human Immunodeficiency virus (HIV). It has further been disclosed as being useful in the treatment of various inflammatory diseases in WO2020/127843**.**

ABX464 is a drug candidate under clinical trials in the treatment of moderate to severe ulcerative colitis (UC).

A route of synthesis of the glucuronide derivative is disclosed in WO2016/135052 according to the route of synthesis as depicted below 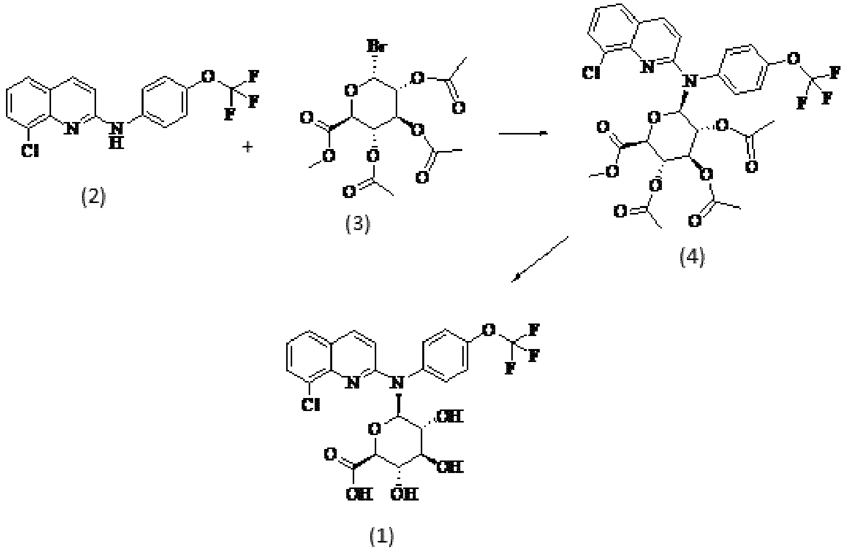 wherein said compound **(4)** is also disclosed as a new intermediate compound.

The reaction between compounds **(2)** and **(3)** may be performed according to a Koenigs-Knorr synthesis. Compound **(4)** may then be thereafter deprotected to afford compound **(1)**.

However, the purification step as disclosed in WO2016/135052, i.e. extraction with ethyl acetate of the crude compound (1), followed by a concentration to dryness to afford a solid extract, is not compliant with the industrial scale. Indeed, it is not desirable implementing a concentration to dryness after said extraction and even not feasible to deal with solid extracts at an industrial scale. The inventors have in other words stated that scale-up generated purification issues.

Therefore, there is a need to provide a process of purification of a quinolinyl-2-yl-phenylalamine glucuronide derivatives of formula (I) as defined herein after compliant with an industrial scale production. There is an additional need to provide a quinolinyl-2-yl-phenylalamine glucuronide derivatives of formula (I) presenting an improved purity, and in particular respecting the ICH level requirements with respect to residual solvent amounts. There is another additional need to provide a manufacturing process for preparing a quinolinyl-2-yl-phenylalamine glucuronide derivatives of formula (I) as defined herein after compliant with industrial implementation with acceptable yield.

Other advantages with respect to WO 2016/135052, in connection to the industrial scale constraint, are detailed herein after.

### SUMMARY OF THE INVENTION

The inventors have surprisingly found specific conditions for purifying the crude quinolinyl-2-yl-phenylalamine glucuronide derivatives of formula (I) as defined herein after, in particular obtained after a deprotection step of compound (IV) as defined herein after. The inventors have in particular developed means for purifying said derivatives of formula (I) starting from a solution phase and not from a solid extract.

The present invention is additionally intended to provide a method for preparing quinolinyl-2-yl-phenylalamine glucuronide derivatives of formula (I) as defined herein after, which is improved in the perspective of an industrial-scale mass production. The inventors have in particular simplified various aspects as it will be more apparent in the following description, which globally renders the claimed process perfectly compliant with the industrial scale.

Herein is thus provided a process of purification of a crude compound of formula (I) 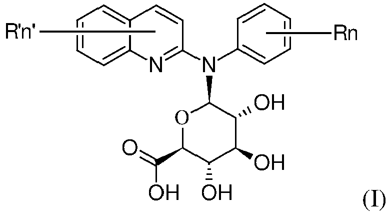 wherein
each **R** independently represents hydrogen, halogen, -CN, hydroxyl, (C₁-C₃)fluoroalkyl, (C₁-C₃)fluoroalkoxy, (C₃-C₆)cycloalkyl, -NO₂, -NR₁R₂, (C₁-C₄)alkoxy, phenoxy, -NR₁-SO₂-NR₁R₂, -NR₁-SO₂-R1, -NR₁C(=O)-R₁, -NR₁-C(=O)-NR₁R₂, -SO₂₋N R₁R₂, -SO₃H, -O-SO₂-OR₃, -O- P(=O)-(OR₃)(OR₄), -O-CH₂-COOR₃, (C₁-C₃)alkyl, said alkyl being optionally mono- or di-substituted by a hydroxyl group, a group of formula (IIa): 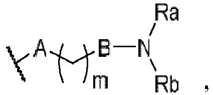
or a group of formula (IIIa) 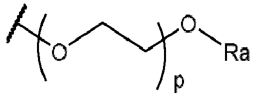 ,
each of R₁ and R₂ is independently hydrogen or (C₁-C₃)alkyl;
each of R₃ and R₄ is independently hydrogen, Li⁺, Na⁺, K⁺, N⁺(Ra)₄ or benzyl;
each **R'** is independently hydrogen, (C₁-C₃)alkyl, hydroxyl, halogen, -NO₂, - NR₁R₂, morpholinyl, morpholino, N-methylpiperazinyl, (C₁-C₃)fluoroalkyl, (C₁-C₄)alkoxy,-O-P(=O)-(OR₃)(OR₄),- CN,
a group of formula (IIa): 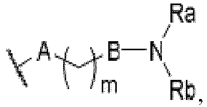
or a group of formula (IIIa): 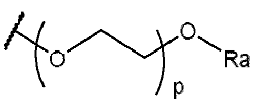
A is a covalent bond, oxygen, or NH;
B is a covalent bond or NH;
m is 1, 2, 3, 4 or 5;
p is 1, 2 or 3; each of Ra and Rb is independently hydrogen, (C₁-C₅)alkyl, or (C₃₋C₆)cycloalkyl, or
Ra and Rb form together with the nitrogen atom to which they are attached a saturated 5- or 6- membered heterocycle, said heterocycle being optionally substituted by one or more Ra, provided that when R' is a group (IIa) or (IIIa), n' may be 2 or 3 only if other R' groups are different from said group (IIa) or (IIIa); and
**n** is 1, 2 or 3;
**n'** is 1, 2 or 3;
wherein it comprises an isolation step of said crude compound of formula (I) including:
   (i) a solubilization step of said crude compound of formula (I) with methanol followed by a precipitation step with water, and
   (ii) a drying step under a temperature inferior or equal to 30°C.

Herein is more particularly provides a process for the purification of a compound of formula (1)

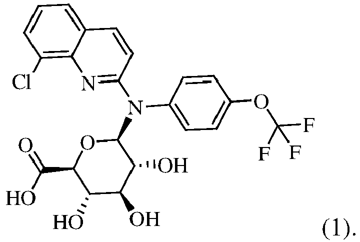

Herein is further provided a process for the manufacture of formula (I) as defined above, wherein it comprises
- a step 1, wherein a compound of formula (II) 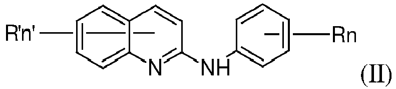 is reacted with a compound of formula (III) 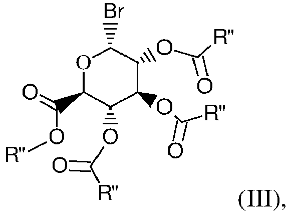 for obtaining a compound of formula (IV) 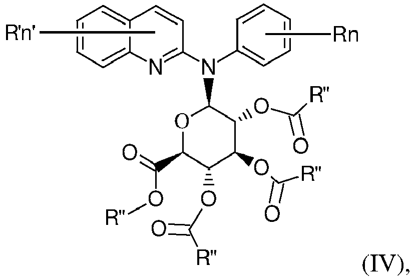 wherein **R**, **R'**, **n** and **n'** are as defined in any of claims 1 to 4 and each **R"** represents a (C₁-C₄)alkyl group, in the presence of CdCO₃, in a solvent selected from aromatic solvents, such as toluene or xylene, in particular in toluene, and with a fractional introduction of the compound of formula (III) in solution, followed by
- a step 2, wherein said compound of formula (IV) is deprotected for obtaining said compound of formula (I) with lithium hydroxide, potassium hydroxide or sodium hydroxide, in particular lithium hydroxide, in absence of hydrogen peroxide and in presence of a solvent selected from aromatic hydrocarbons such as toluene or xylene; ethers, such as tetrahydrofuran or methyl tert-butyl ether (MTBE), in particular tetrahydrofuran, at room temperature.

As used herein, the term "ambient temperature" or "room temperature" refers to a temperature ranging from 15°C to 30°C, more particularly from 18°C to 25°C.

### DETAILED DESCRIPTION OF THE INVENTION

As it will be more detailed herein after, the isolation of the desired product is achieved through precipitation, which may be followed by a purification step on silica gel chromatography column, itself optionally preceded by a silica gel pre-treatment.

According to one embodiment, R, n, R' and n' have the following meanings:
Each **R** independently represent a hydrogen atom, a halogen atom or a group chosen among a -CN group, a hydroxyl group, a -COOR₁ group, a (C₁-C₃)fluoroalkyl group, a (C₁-C₃)fluoroalkoxy group, a -NO₂ group, a -NR₁R₂ group, a (C₁-C₄)alkoxy group, a phenoxy group and a (C₁-C₃)alkyl group, said alkyl being optionally mono-substituted by a hydroxyl group,
R₁ and R₂ are independently a hydrogen atom or a (C₁-C₃)alkyl group,
**n** is 1, 2 or 3,
**n'** is 1 or 2, and
each **R'** independently represents a hydrogen atom or a group chosen among a (C₁-C₃)alkyl group, a halogen atom, a hydroxyl group, a -COOR₁ group, a -NO₂ group, a -NR₁R₂ group, a morpholinyl or a morpholino group, a N-methylpiperazinyl group, a (C₁-C₃)fluoroalkyl group, a (C₁-C₄)alkoxy group and a -CN group.

According to another embodiment, R, n, R' and n' have the following meanings:
Each **R** independently represent a hydrogen atom, a halogen atom or a group chosen among a (C₁-C₃)fluoroalkyl group, a (C₁-C₃)fluoroalkoxy group, a -NR₁R₂ group, a (C₁-C₄)alkoxy group, a phenoxy group and a (C₁-C₃)alkyl group, said alkyl being optionally mono-substituted by a hydroxyl group,
R₁ and R₂ are independently a (C₁-C₃)alkyl group,
**n** is 1, 2 or 3, preferably 1 or 2, an even more preferably 1,
**n'** is 1 or 2, preferably 1, and
each **R'** independently represents a hydrogen atom or a group chosen among a (C₁-C₃)alkyl group, a halogen atom, a -NR₁R₂ group, a (C₁-C₃)fluoroalkyl group and a (C₁-C₄)alkoxy group.

According to another embodiment, the compound of formula (I) is

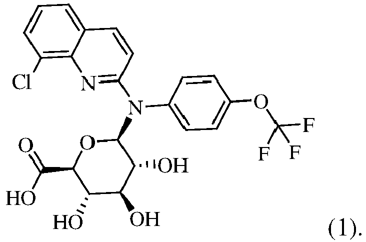

In the context of the present invention, the term:
- "halogen" is understood to mean chlorine, fluorine, bromine, or iodine, and in particular denotes chlorine, fluorine or bromine,
- "(C₁-Cₓ)alkyl" as used herein respectively refers to C₁-Cₓ normal, secondary or tertiary saturated hydrocarbon. Examples are, but are not limited to, methyl, ethyl, 1-propyl, 2-propyl, butyl, pentyl,
- "(C₃-C₆)cycloalkyl" as used herein respectively refers to cyclic saturated hydrocarbon.

Examples are, but are not limited to cyclopropyl, cyclobutyl, cyclopentyl, cyclohexyl,
- "(C₁-C₄)alkoxy" as used herein respectively refers to O-(C₁-C₄)alkyl moiety, wherein alkyl is as defined above. Examples are, but are not limited to, methoxy, ethoxy, 1-propoxy, 2-propoxy, butoxy,
- "fluoroalkyl group" and "fluoroalkoxy group" refers respectively to alkyl group and alkoxy group as above-defined, said groups being substituted by at least one fluorine atom. Examples are perfluoroalkyl groups, such as trifluoromethyl or perfluoropropyl,
- "saturated 5- or 6-membered heterocycle" as used herein respectively refers to a saturated cycle comprising at least one heteroatom. Examples are, but are not limited to, morpholine, piperazine, thiomorpholine, piperidine, pyrrolidine.

### Purification method/step

The purification step as disclosed in WO2016/135052 consists in an extraction step with ethyl acetate of the crude compound (1) after having quenched the reaction mixture which is then acidified, followed by an extraction with ethyl acetate and a drying step on sodium sulfate and at last purification on silica column of the solid extract obtained after having removed the solvent.

The inventors have stated that this procedure was not compliant with the industrial scale in particular due to the huge difficulties the implementation of solid dry extracts involves at the industrial scale.

Thus, they have developed an isolation step allowing to comply with handling a solution or suspension instead of a solid concentrate and an acceptable purity requirement of said compound (1) as well as acceptable level of residual solvent, according to the common standards in the pharmaceutical industry, namely ICH Residual solvents requirements of the European Medicines Agency. The inventors thus found out that the use of a particular solvent precipitation system at a particular temperature, which may be preceded by a purification step on silica gel column, itself optionally preceded by a silica gel pre-treatment, allowed to meet these expectations.

Indeed, the inventors have stated that undesired residual solvent amounts after precipitation could be detected and at the same time, potential degradation under heating conditions when attempting to eliminate said residual solvents occurred.

Faced with this technical problem, the inventors have found out that a precipitation solvent system methanol with water is more particularly suitable for reaching suitable yield and solvent removal.

They have moreover established that a drying temperature inferior or equal to 30°C is more particularly recommended for avoiding the degradation of compound (1).

According to a particular embodiment, the drying step may be performed at a temperature ranging from 20 to 30°C.

The crude compound of formula (I), and in particular crude compound (1), in particular the reaction mixture obtained after a deprotection step of compound (IV) as defined herein after, can therefore be diluted with methanol, and after the addition over cold water, a brown solid precipitate may be obtained.

As apparent in example 2 herein after, significant improvements in terms of acceptable amount of residual solvent have been obtained by using said particular solvent system for the precipitation and by drying at a temperature inferior or equal to 30°C.

Still as apparent in example 2 herein after, residual amounts of toluene, methylene chloride, methanol and tetrahydrofuran may be obtained, compliant with the ICH levels requirements. Namely, ICH level requirement for methanol is under 3000 ppm with respect to the total amount of the desired compound. Similarly, ICH level requirement for tetrahydrofuran is 720 ppm with respect to the total amount of the desired compound. Similarly, ICH level requirement for toluene is 890 ppm with respect to the total amount of the desired compound. Similarly, ICH level requirement for methylene chloride is 600 ppm with respect to the total amount of the desired compound.

According to one aspect, herein is provided a process of purification of a crude compound of formula (I) as defined above wherein it is preceded by a purification step on silica gel column, optionally preceded by a silica gel pre-treatment.

Herein is thus provided a process of purification as previously disclosed, wherein it is preceded by a purification on silica gel chromatography column, in particular eluted with methylene chloride, methanol or mixture thereof, optionally preceded by a silica gel pre-treatment.

During the optional pre-treatment by a silica gel, said compound of formula (I) as defined above is adsorbed onto the silica gel. Said optional preliminary stage of purification allows avoiding having a greater number of runs on the silica gel column for obtaining the compound of formula (I). Said silica gel pre-treatment also presents the advantage of allowing to gather the unreacted compound of formula (II), in particular of formula (2), which may be reused for further manufacturing batches for preparing derivatives of formula (I), in particular of formula (1).

Silica gel may be washed at a first time with a solvent selected from methylene chloride, tetrahydrofuran or mixture thereof. The number of washes of these first series of washes may vary between 1 and 3. The product may then be desorbed with a second washing sequence with only THF or only methanol. The number of washes of these second series of washes may vary between 1 and 3.

As far as the silica gel chromatography step is concerned, the silica gel column may in particular be selected from normal phase and in particular from silica gel "Chromatorex GS60-20/45". The elution may be performed with a methylene chloride/methanol mixture.

Implementing a further process of purification according to the present invention allowed to reach an overall yield of compound (1) ranging from 10% to 20%, in particular from 11% to 17%.

All in all, the purification step as described above allows obtaining a compound of formula (I) presenting good quality properties and namely a high purity, and also presenting acceptable levels of residual solvents.

Among the impurities the inventors have detected in the final compound of formula (I), in particular of formula (1), the starting compound of formula (II) may be cited as well as the methyl ester having the following formula (V) 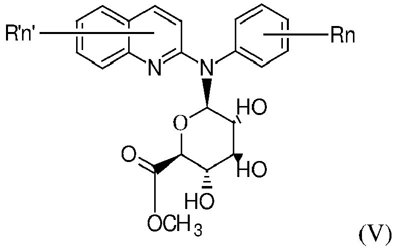 which for compound (1) is compound (5)

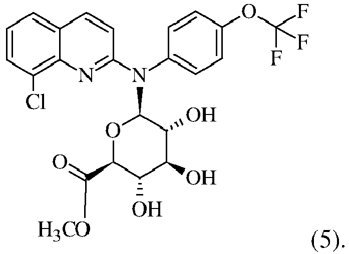

The compound of formula (I) obtained according to the present invention, and in particular compound (1) may typically present a purity ranging from 90 to 97%, in particular from 92 to 96%, and even more particularly from 93 to 96%, with an amount of compound of formula (II), in particular compound (2) ranging from 0.3 to 2.1% and an amount of impurity of formula (V), in particular impurity (5) ranging from 1.9 to 2.3%.

The compound of formula (I) obtained according to the present invention, and in particular compound (1) may typically present residual content of methanol inferior to 750ppm, methylene chloride inferior to 420ppm, tetrahydrofuran inferior to 360ppm and toluene inferior to 445ppm.

### Method of preparation of compound of formula (I)

According to one embodiment, the present process of purification may be carried out after a deprotection step of a compound of formula (IV) 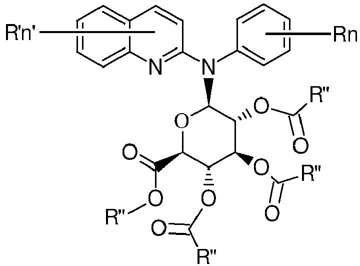 (IV), wherein R, R', n, n' and R" are as defined above, as more detailed herein after as step 2.

Compound of formula (IV) may be prepared by reacting a compound of formula (II) 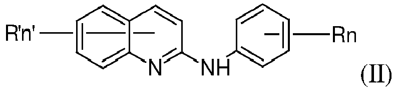 with a compound of formula (II) 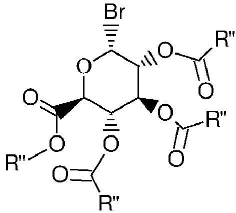 (III), wherein R" is as defined above, according to a Koenigs-Knorr synthesis as more detailed herein after as step 1.

Herein is further provided an improved process for preparing a compound of formula (I) as defined above, implementing step 1 and step 2 as defined herein after, before the purication of the crude compound of formula (I) as provided herein.

The manufacturing process disclosed in WO2016/135052 for obtaining chloro-N-[4-(trifluoromethoxy)phenyl]quinolin-2-amine glucuronide in an amount of less than 5 g, which is not applicable as long as a scale up is envisioned.

### Step 1

As more detailed herein after, step 1 differs from said coupling step as disclosed in WO2016/135052 mainly by the introduction of the compound of formula (III) in solution and with a fractional introduction.

Further improvements may further be implemented. For example, the 12 hours reflux phase before introduction of compound (III) may be suppressed. Further, the purification step on silica gel chromatography may be avoided. In addition, the obtained crude product (IV), in particular compound (4) may be maintained in solution in a solvent such as THF rather than worked-up as a viscous dry extract. At last, solvent volumes may be decreased for increasing the productivity.

The conversion rate of compound of formula (II) as defined above, into compound of formula (IV) is quite low. For example, rework of WO2016/135052 could give around 50% of remaining compound (2) even after 5 days of reaction. It was moreover stated that compound (3) could degrade which strongly impacts the overall yield of the reaction.

In accordance with the present invention, step 1 may be implemented by reacting compound of formula (II) with compound of formula (III) as defined above, in the presence of CdCO₃, in a solvent selected from aromatic solvents, such as toluene or xylene, more particularly in toluene.

Herein is provided an improved process for preparing a compound of formula (IV), wherein compound of formula (III) is reacted in a molar ratio with respect to the compound of formula (II) ranging from 1.2 to 3, in particular from 1.4 to 2.5, and even more particularly from 1.5 to 1.7, in the presence of toluene, in a volume ratio with respect to the compound of formula (II) ranging from10 to 30, in particular from 10 to 20, and more particularly from 10 to 15.

The reaction may be performed at reflux temperature, i.e. at 111°C.

The reduction of volume of solvent allowed to increase the conversion rate.

The inventors have moreover stated that a fractional introduction of the compound of formula (III) in solution allowed to improve the reaction conversion.

Thus, according to a particular embodiment, step 1 may be carried out with a fractional introduction of the compound of formula (III) in solution, in particular of compound (3), in particular in two parts. Still according to said particular embodiment, a first introduction may be performed with a molar ratio of compound of formula (III) with respect to the compound of formula (II), in particular of compound (3) with respect to compound (2), ranging from 0.6 to 1, in particular ranging from 0.7 to 0.9, and a second introduction may be performed with a molar ratio of compound of formula (III) with respect to the compound of formula (II), in particular of compound (3) with respect to compound (2), ranging from 0.6 to 1, in particular ranging from 0.7 to 0.9, with a total molar ratio with respect to compound of formula (II) ranging from 1.2 to 2, in particular from 1.4 to 1.8.

Step 1 may be carried out for 10 to 120 hours, in particular for 20 to 80 hours, and even more particularly for 30 to 50 hours.

Yield at this stage of the manufacturing process is quantitative.

Example 1.1 more particularly illustrates said step 1.

### Step 2

As more detailed herein after, step 2 differs from the deprotection step as disclosed in WO2016/135052 mainly by the removal of hydrogen peroxide. Said removal presents an advantage in terms of security of the process.

Further improvements may further be implemented. For example, the present step 2 does not need anymore the use of sodium thiosulfate. At last, solvent volumes, such as of THF and of water may be decreased. The isolation step and work-up improvements with the isolation by precipitation with a mixture of methanol and water has already been described above, as well as the implementation of an optional pre-treatment step on silica gel.

As apparent in WO2016/135052, compound (4) was reacted in a second step in presence of hydrogen peroxide in a suspension of lithium hydroxide monohydrate. The inventors have stated that the presence of hydrogen peroxide is in fact not essential for this deprotection step.

Herein is provided an improved process for preparing a compound of formula (I) as defined above from compound of formula (IV) as defined above, wherein compound of formula (IV) is reacted with lithium hydroxide, potassium hydroxide or sodium hydroxide, in particular lithium hydroxide in presence of a solvent selected from aromatic hydrocarbons such as toluene or xylene; ethers, such as tetrahydrofuran or methyl tert-butyl ether (MTBE); in particular tetrahydrofuran, at room temperature.

The solvent may be implemented in a volume ratio with respect to compound of formula (IV) ranging from 5 to 30, in particular from 5 to 15, more particularly from 5 to 7.

Yield at this stage of the manufacturing process, i.e. after the purification step, including the hereabove described pre-treatment on silica gel, the purification on silica gel column and the precipitation step as described above, may range from 10% to 20%, in particular from 12% to 17%.

Example 1.2 more particularly illustrates said step 2.

Thus, herein is further provided a process of purification according to the present invention, wherein it is preceded by a deprotection step of a compound of formula (IV) 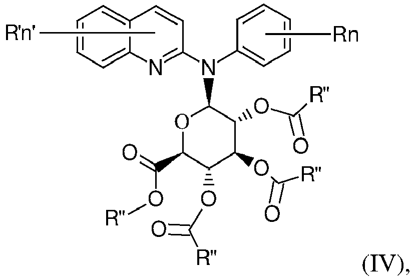 wherein **R**, **R'**, **n** and **n'** are as defined in any of claims 1 to 4 and each **R"** represents a (C₁-C₄)alkyl group, for obtaining said compound of formula (I) with lithium hydroxide , potassium hydroxide or sodium hydroxide, in particular lithium hydroxide, in presence of a solvent selected from aromatic hydrocarbons such as toluene or xylene; ethers, such as tetrahydrofuran or methyl tert-butyl ether (MTBE); in particular tetrahydrofuran, at room temperature.

According to one variant, **R"** is a methyl group.

According to another variant, the process of purification according to the preceding claim, wherein said compound of formula (IV) is compound (4)

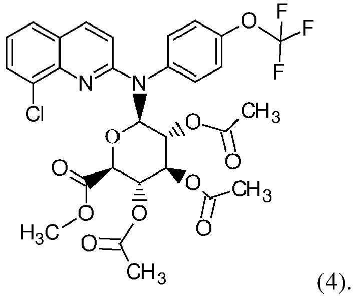

Herein is further provided a process of purification according to the present invention, wherein said deprotection step is preceded by a step of reaction of a compound of formula (II) 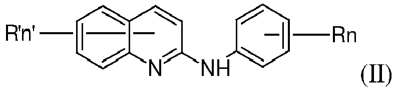 with a compound of formula (III) 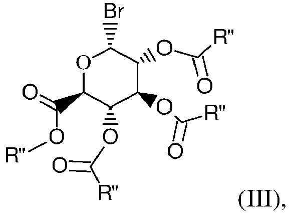 for obtaining a compound of formula (IV) 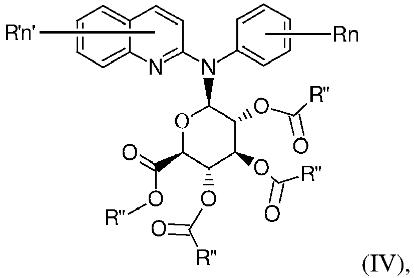 wherein **R**, **R'**, **n** and **n'** are as defined in any of claims 1 to 4 and each **R"** represents a (C₁-C₄)alkyl group, in particular a methyl group, in the presence of CdCO₃, in a solvent selected from aromatic solvents, such as toluene or xylene, in particular in toluene, and with a fractional introduction of the compound of formula (III) in solution.

According to one variant, the compound of formula (II) is compound (2) 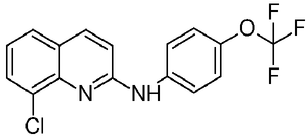 (2), and the compound of formula (III) is compound (3)

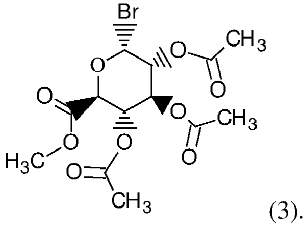

Herein is also provided a novel process for the preparation of compound (1) wherein it comprises the step of implementing step 1 and 2 as described above, and more particularly a manufacturing process for preparing a compound of formula (I) as above, wherein it comprises
- a step 1, wherein a compound of formula (II) 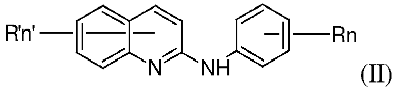 is reacted with a compound of formula (III) 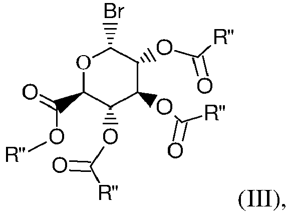 for obtaining a compound of formula (IV) 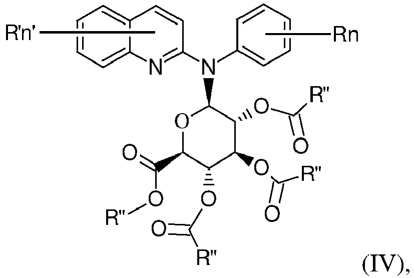 wherein **R**, **R'**, **n** and **n'** are as defined in any of claims 1 to 4 and each **R"** represents a (C₁-C₄)alkyl group, in the presence of CdCO₃, in a solvent selected from aromatic solvents, such as toluene or xylene, in particular in toluene, and with a fractional introduction of the compound of formula (III) in solution, followed by
a step 2, wherein said compound of formula (IV) is deprotected for obtaining said compound of formula (I) with lithium hydroxide, potassium hydroxide or sodium hydroxide, in particular lithium hydroxide, in absence of hydrogen peroxide and in presence of a solvent selected from aromatic hydrocarbons such as toluene or xylene; ethers, such as tetrahydrofuran or methyl tert-butyl ether (MTBE), in particular tetrahydrofuran, at room temperature.

Herein is further provided a process for preparing a compound of formula (I) as define above, in particular compound (1) as defined above, further comprising the step of preparing a pharmaceutical composition containing such compound of formula (I), in particular compound (1), with pharmaceutically acceptable excipients.

Throughout the description, including the claims, the term "comprising a" should be understood as being synonymous with "comprising at least one", unless otherwise specified.

The expressions "between ... and ...", and "ranging from ... to ..." should be understood as meaning limits included, unless otherwise specified.

Hereinafter, the present invention will be described in more detail with reference to the following examples. These examples are provided to illustrate the present invention and should not be construed as limiting the scope and spirit of the present invention.

### EXAMPLES

### List of abbreviations

- Methylene chloride: DCM,
- Tetrahydrofuran: THF.

### Example 1: Manufacturing process for preparing crude compound (1)

### 1.1 Step 1

### 1.1.1 With solid fractional introduction of (3) (comparative)

A compound (2) as defined above (20.0g) was mixed with cadmium carbonate (6.0g, 0.6 eq) and toluene (580mL, 29 volumes). The resulting mixture was azeotropically dried for 1h before compound (3) as defined above (38.0g, 1.6 eq.) was added in four portions over 28h onto the refluxing mixture. The mixture was heated at reflux for an additional period of 46h before being cooled down to room temperature, filtered and rinsed with methylene chloride. Filtrates were joined and concentrated to dryness before being diluted in DCM, washed 3 times with water, dried over sodium sulfate, concentrated to dryness and diluted in THF before being engaged in step 2.

Compound (4) was isolated with a quantitative yield and an HPLC purity of 43% (53% of remaining compound (2)).

### 1.1.2 With fractional introduction of (3) in solution and decrease of the dilution (invention)

Said compound (2) (65.0kg) was mixed with cadmium carbonate (52.7kg, 1.6 eq) and toluene (325L, 5 volumes). The resulting mixture was heated to reflux for azeotropic drying and a solution of compound (3) (61.1kg, 0.8 eq.) in toluene (318.5L, 2.5 volumes) was added at this temperature over 8h. Partial distillation of toluene (160L, 2.5 vol) was performed and the reflux was maintained during the night (10h). The second part of the solution of compound (3) (61.1kg, 0.8 eq.) in toluene (318.5L, 2.5 volumes) was added at reflux over 8.5h. Partial distillation of toluene (160L, 2.5 vol) was performed and the reflux was maintained during 24h. The reaction mixture was then cooled down to room temperature, filtered and rinsed with methylene chloride. Filtrates were joined and washed with an aqueous solution of sodium bicarbonate (5.2kg in 65L of water) followed by water (65L). The resulting organic layer was then concentrated to dryness and the residue diluted in THF (130L) before being engaged in step 2.

Compound (4) was isolated with a quantitative yield (126kg) and an HPLC purity of 48%.

It is thus herein confirmed that a fractional introduction of the compound (3) allows to afford compound (4) also with quantitative yield and with a slightly improved purity.

### 1.2 Step 2

Said compound (4) in THF solution resulting from step 1 (310kg of solution, 126kg of (4)) was diluted with tetrahydrofuran (449kg). A solution of lithium hydroxide monohydrate (80.6kg) in water (327L) was added at 20°C onto the compound (4) solution and stirred for 2h at 20°C. Aqueous layer was discarded and the organic layer was concentrated, diluted with water (252L) and methylene chloride (670kg) and acidified until pH 1 with hydrochloric acid. The organic layer was separated and the aqueous layer extracted with DCM (335kg). Organic layers were joined and washed with water (252L). Silica gel (90.8kg) was added to the organic layer, the mixture stirred for 30min at 20°C, filtered and the silica gel pad washed with methylene chloride (168kg) and twice with a mixture of DCM (160kg) and THF (6.2L). These three washes were joined for a further valorisation of unreacted compound (2). The silica gel pad was then washed successively with 112kg and 2x56kg of THF. The resulting solution was concentrated under vacuum and the residue diluted in a mixture of DCM (67kg) and THF (1.3L). The resulting solution was purified by silica gel chromatography with a DCM/methanol mixture as eluant. Collected fractions were gathered, concentrated under vacuum and diluted in 1 volume of methanol (15L) with respect to estimated amount of compound (1).

### Example 2: Process of purification of a crude compound (1)

### 2.1.With a methanol/water system and drying at 30°C (invention)

A precipitation of crude compound (1) as produced in example 1 is performed as follows.

Said crude compound (1) (15kg) diluted in 1 vol of methanol (15L) from example 1 was then added on 20 volumes of demineralised water (300L) at 0/5°C over 1 hour and rinsed with 0.5 volume of methanol. The suspension was filtered and the cake washed with demineralised water (15L). The wet product was dried in tray oven at 30°C until water content and residual solvents were meeting the specifications. The obtained product was a brown solid obtained with 95% of yield (14.35kg).

Compound (1) obtained according to the present protocol have a 95.3% HPLC purity and contains 0.8% of compound (2) and 2.0% of methyl ester impurity (5). Water content was 3.5% and residual solvents were as follow: methanol < 750ppm, DCM < 150ppm, THF < 180ppm and toluene <223pm.

The purification step performed according to the present invention has been observed as being completely compliant with the industrial scale, and affords compound (1), under an amorphous solid form, respecting the ICH Residual solvents requirements of the European Medicines Agency.

### 2.2.With a methanol/water system and drying at 50°C (comparison)

A part partitioned as in example 2.1 above was dried at 30°C for 100h and at 50°C during 11 hours.

The obtained product was a brown solid with an HPLC purity of 93.4% and contains 2.1% of compound (2) and 2.1% of methyl ester impurity (5).

Water content was 3.2% and residual solvents were as follow: methanol 738ppm, DCM <300ppm, THF <360ppm and toluene <445pm.

The purification step performed according to a process outside the present invention (i.e. with a temperature above 30°C) has been observed as conducting to much more undesirable degradation.

### 2.3.With a DCM/heptane system (comparison)

A precipitation of crude compound (1) as produced in example 1 is performed as follows.

Said crude compound (1) is solubilized (1.4g) with 4 vol of DCM and 0.6 vol of THF to obtain a solution. Said solution is added onto 10 vol of heptane (with 0.5 of IPA) over 1h45 at 20-25°C, and then rinsed with 0.5 vol of DCM. The mixture is then stirred for 2h30 at room temperature. Filtration of the fluid suspension is then carried out and washed with 4^{∗}0.5 vol of heptane to give 0.856 g of wet brown product.

Said product is dried under vacuum at 20°C for 16.5h and after at 50° for 4 days. The obtained product was a brown solid obtained with 77% of yield.

Isolated compound (1) has an HPLC purity of 89.4% (1.8% of methyl ester impurity (5) and 4.6% of compound (2)) and the following residual solvent contents: methanol = 782ppm, THF = 6157ppm, toluene = 59ppm and heptane = 64000ppm.

The purification step performed according to a process outside the present invention (i.e. with another solvent system) has been observed as affording compound (1) with less yield and with a lower purity as under the conditions conform the present invention. Furthermore, the ICH Residual solvents requirements of the European Medicines Agency are not fulfilled.

## Claims

1. A process of purification of a crude compound of formula (I) 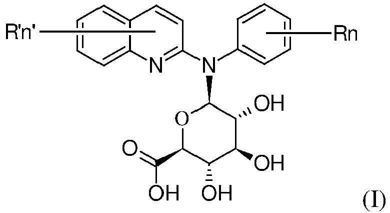 wherein
each **R** independently represents hydrogen, halogen, -CN, hydroxyl, (C₁-C₃)fluoroalkyl, (C₁-C₃)fluoroalkoxy, (C₃-C₆)cycloalkyl, -NO₂, -NR₁R₂, (C₁-C₄)alkoxy, phenoxy, -NR₁-SO₂-NR₁R₂, -NR₁-SO₂-R1, -NR₁C(=O)-R₁, -NR₁-C(=O)-NR₁R₂, -SO₂₋N R₁R₂, -SO₃H, -O-SO₂-OR₃, -O- P(=O)-(OR₃)(OR₄), -O-CH₂-COOR₃, (C₁-C₃)alkyl, said alkyl being optionally mono- or di-substituted by a hydroxyl group, a group of formula (IIa): 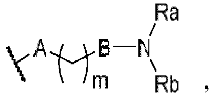
or a group of formula (IIIa) 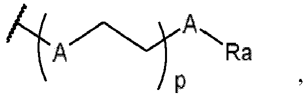
each of R₁ and R₂ is independently hydrogen or (C₁-C₃)alkyl;
each of R₃ and R₄ is independently hydrogen, Li⁺, Na⁺, K⁺, N⁺(Ra)₄ or benzyl;
each **R'** is independently hydrogen, (C₁-C₃)alkyl, hydroxyl, halogen, -NO₂, - NR₁R₂, morpholinyl, morpholino, N-methylpiperazinyl, (C₁-C₃)fluoroalkyl, (C₁-C₄)alkoxy,-O-P(=O)-(OR₃)(OR₄),- CN,
a group of formula (IIa): 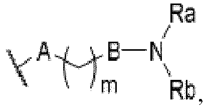
or a group of formula (IIIa): 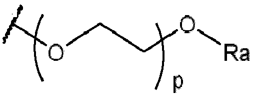
A is a covalent bond, oxygen, or NH;
B is a covalent bond or NH;
m is 1, 2, 3, 4 or 5;
p is 1, 2 or 3; each of Ra and Rb is independently hydrogen, (C₁-C₅)alkyl, or (C₃₋C₆)cycloalkyl, or
Ra and Rb form together with the nitrogen atom to which they are attached a saturated 5- or 6- membered heterocycle, said heterocycle being optionally substituted by one or more Ra, provided that when R' is a group (IIa) or (IIIa), n' may be 2 or 3 only if other R' groups are different from said group (IIa) or (IIIa); and
**n** is 1, 2 or 3;
**n'** is 1, 2 or 3;
wherein it comprises an isolation step of said crude compound of formula (I) including:
(i) a solubilization step of said crude compound of formula (I) with methanol followed by a precipitation step with water, and
(ii) a drying step under a temperature inferior or equal to 30°C.

2. The process of purification of a crude compound of formula (I) according to claim 1, wherein
each **R** independently represent a hydrogen atom, a halogen atom or a group chosen among a -CN group, a hydroxyl group, a -COOR₁ group, a (C₁-C₃)fluoroalkyl group, a (C₁-C₃)fluoroalkoxy group, a -NO₂ group, a -NR₁R₂ group, a (C₁-C₄)alkoxy group, a phenoxy group and a (C₁-C₃)alkyl group, said alkyl being optionally mono-substituted by a hydroxyl group,
R₁ and R₂ are independently a hydrogen atom or a (C₁-C₃)alkyl group,
**n** is 1, 2 or 3,
**n'** is 1 or 2, and
each **R'** independently represents a hydrogen atom or a group chosen among a (C₁-C₃)alkyl group, a halogen atom, a hydroxyl group, a -COOR₁ group, a -NO₂ group, a -NR₁R₂ group, a morpholinyl or a morpholino group, a N-methylpiperazinyl group, a (C₁-C₃)fluoroalkyl group, a (C₁-C₄)alkoxy group and a -CN group.

3. The process of purification of a crude compound of formula (I) according to claim 1 or 2, wherein
each **R** independently represent a hydrogen atom, a halogen atom or a group chosen among a (C₁-C₃)fluoroalkyl group, a (C₁-C₃)fluoroalkoxy group, a -NR₁R₂ group, a (C₁-C₄)alkoxy group, a phenoxy group and a (C₁-C₃)alkyl group, said alkyl being optionally mono-substituted by a hydroxyl group,
R₁ and R₂ are independently a (C₁-C₃)alkyl group,
**n** is 1, 2 or 3, preferably 1 or 2, an even more preferably 1,
**n'** is 1 or 2, preferably 1, and
each **R'** independently represents a hydrogen atom or a group chosen among a (C₁-C₃)alkyl group, a halogen atom, a -NR₁R₂ group, a (C₁-C₃)fluoroalkyl group and a (C₁-C₄)alkoxy group.

4. The process of purification of a crude compound of formula (I) according to any of claims 1 to 3, wherein the compound of formula (I) is

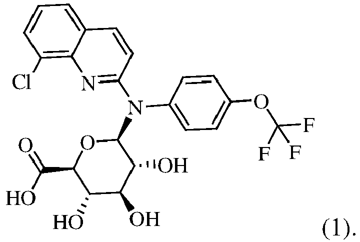

5. The process of purification of a crude compound of formula (I) according to any of claims 1 to 4, wherein it is followed by a purification on silica gel chromatography column, in particular eluted with methylene chloride, methanol or mixture thereof, optionally preceded by a silica gel pre-treatment.

6. The process of purification according to any of the preceding claims, wherein it is preceded by a deprotection step of a compound of formula (IV) 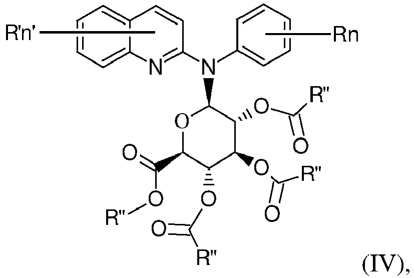 wherein **R**, **R'**, **n** and **n'** are as defined in any of claims 1 to 4 and each **R"** represents a (C₁-C₄)alkyl group, for obtaining said compound of formula (I) with lithium hydroxide , potassium hydroxide or sodium hydroxide, in particular lithium hydroxide, in presence of a solvent selected from aromatic hydrocarbons such as toluene or xylene; ethers, such as tetrahydrofuran or methyl tert-butyl ether (MTBE); in particular tetrahydrofuran, at room temperature.

7. The process of purification according to the preceding claim, wherein said compound of formula (IV) is compound (4)

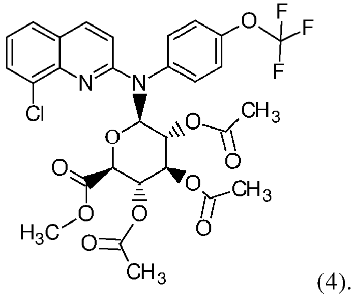

8. The process of purification according to claim 6 or 7, wherein said deprotection step is preceded by a step of reaction of a compound of formula (II) 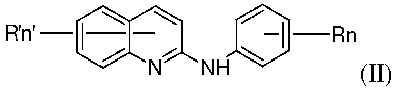 with a compound of formula (III) 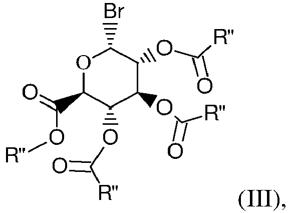 for obtaining a compound of formula (IV) 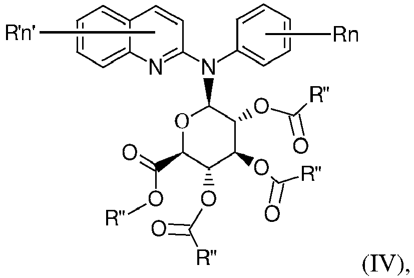 wherein **R**, **R'**, **n** and **n'** are as defined in any of claims 1 to 4 and each **R"** represents a (C₁-C₄)alkyl group, in particular a methyl group, in the presence of CdCO₃, in a solvent selected from aromatic solvents, such as toluene or xylene, in particular in toluene, and with a fractional introduction of the compound of formula (III) in solution.

9. The process according to the preceding claim, wherein the compound of formula (II) is compound (2) 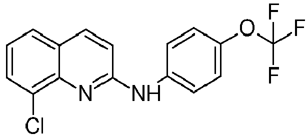 (2), and the compound of formula (III) is compound (3)

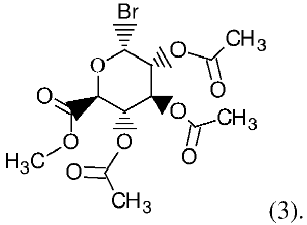

10. The process of purification according to the preceding claim, wherein the fractional introduction of the compound of formula (III) in solution comprises 2 independent additions of the compound of formula (III) in solution, with a first introduction being performed with a molar ratio of compound of compound of formula (III) with respect to the compound of formula (II), ranging from 0.6 to 1, in particular ranging from 0.7 to 0.9, and a second introduction being performed with a molar ratio of compound of formula (III) with respect to the compound of formula (II), ranging from 0.6 to 1, in particular ranging from 0.7 to 0.9, with a total molar ratio with respect to compound of formula (II) ranging from 1.2 to 2, in particular from 1.4 to 1.8

11. A manufacturing process for preparing a compound of formula (I) as defined in any of claims 1 to 4, wherein it comprises
• a step 1, wherein a compound of formula (II) 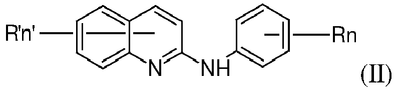 is reacted with a compound of formula (III) 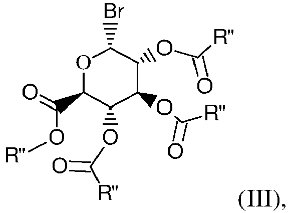 for obtaining a compound of formula (IV) 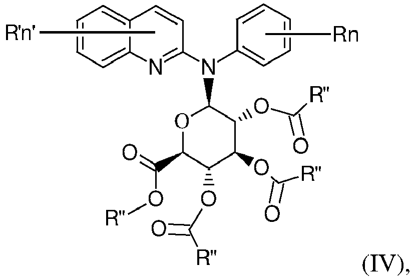 wherein **R**, **R'**, **n** and **n'** are as defined in any of claims 1 to 4 and each **R"** represents a (C₁-C₄)alkyl group, in the presence of CdCO₃, in a solvent selected from aromatic solvents, such as toluene or xylene, in particular in toluene, and with a fractional introduction of the compound of formula (III) in solution, followed by
• a step 2, wherein said compound of formula (IV) is deprotected for obtaining said compound of formula (I) with lithium hydroxide, potassium hydroxide or sodium hydroxide, in particular lithium hydroxide, in absence of hydrogen peroxide and in presence of a solvent selected from aromatic hydrocarbons such as toluene or xylene; ethers, such as tetrahydrofuran or methyl tert-butyl ether (MTBE), in particular tetrahydrofuran, at room temperature.

12. The process for preparing a compound of formula (I) as defined in any of claims 1 to 4, further comprising the step of preparing a pharmaceutical composition containing such compound of formula (I), with pharmaceutically acceptable excipients.
